# EUROPEAN PATENT APPLICATION

(11) **EP 1 876 185 A1**
(43) Date of publication of application: **09.01.2008**
(21) Application number: 07111055.5
(22) Date of filing: 26.06.2007
(51) Int. Cl.: C07K 16/18, G01N 33/577, C12N 5/18

(54) **An antibody which recognizes phosphorylated polypeptides**

(30) Priority: 04.07.2006 EP 06116550
(71) Applicant: F. HOFFMANN-LA ROCHE AG, 4070 Basel (CH)
(72) Inventor: Bohrmann, Bernd, 4125 Riehen (CH); Grueninger, Fiona, 4144 Arlesheim (CH); Czech, Christian, 79639 Grenzach-Whylen (DE); Gerlach-Weck, Judith, 55270 Zornheim (DE)
(74) Representative: Küng, Peter

(57) **Abstract**

The present invention relates to an antibody which recognizes an epitope consisting of Ser-Ile-A1-A2-A3-A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5 , wherein A1 is Asp or Asn, A2 is Met or Leu, A3 is Val or Leu, A4 is Asp or Glu and A5 is Asp or Glu, a hybridoma producing the antibody, a kit comprising the antibody, and a method for diagnosis of a neurological disorder.

## Description

The present invention relates to an antibody which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5 , wherein A1 is Asp or Asn, A2 is Met or Leu, A3 is Val or Leu, A4 is Asp or Glu and A5 is Asp or Glu.

Alzheimer's Disease (AD) is the most common form of adult-onset dementia. There is currently no reliable biochemical test or biomarker available for diagnosis of AD and conclusive diagnosis can only be made post-mortem.

Neuropathological examination of post-mortem brain shows large amounts of extracellular amyloid plaques and intracellular neurofibrillary tangles (NFTs) in characteristic regions of the brain. NFT deposition correlates better with disease severity than plaque accumulation.

NFTs comprise paired helical filaments (PHFs) of the microtubule-associated protein tau (Delacourte, A, J., Neurol. Sci. 76 (1986) 173-180, Kosik, K., PNAS 83 (1986) 4044-4048, Kondo, J., Neuron 1 (1988) 82, Wood, J., PNAS 83 (1986) 4040-4043). The function of tau *in vivo* is to promote microtubule assembly and stability within the axonal compartment of neurons (Lewis, S., Nature 342 (1989) 498-505).

Tau is a highly soluble protein, with no propensity whatsoever to aggregate into filaments. However, in AD, tau becomes hyperphosphorylated. This hyperphosphorylation initiates an aggregation processes which culminates in PHFs and ultimately NFTs. Phosphorylation is a normal feature of tau and is part of the control mechanism which regulates the binding to microtubules (Lovestone, S., Biol. Psychiatry 45(1999) 995-1003).

There is a large number of potential phosphorylation sites within the molecule, these occur within two regions which flank the microtubule binding domain of tau. Phosphorylation at these sites is benign and does not lead to tau aggregation. In AD, a discrete number of additional phosphoepitopes (epitopes comprising at least one phosphorylated amino acid) are found and it is this *hyperphosphorylation* or *abnormal* KM/27.04.2007 phosphorylation which most likely initiates the fibrillization process. An understanding of the mechanisms that lead to PHF-tau formation therefore requires knowledge of these phosphorylation sites

The abnormal phosphoepitopes in tau that occur in AD have been identified by isolation and characterization of PHFs from AD brain (Hasegawa, M., J. Biol. Chem. 267 (1992) 17047-17054, Cripps, D., J. Biol. Chem. 281 (2006) 10825-10838). One of these phosphoepitopes occurs at Ser422, towards the C-terminus of tau. Phosphorylation of Ser422 is tightly linked to NFT formation (Jicha, G., J. Neurochem 69 (1997) 2087-2095). This group showed in cell culture experiments that phosphorylation of tau at Ser422 causes a decrease in tau solubility, implying a role in the initial steps of the aggregation process. Mutation of Ser422 to alanine abolished this effect (Ferrari, A., J. Biol. Chem. 278 (2003) 40162-40168).

Phosphorylation of tau at Ser422 may be a sensitive marker for AD, as suggested by an early study using a polyclonal antibody directed against this phosphoepitope (Bussiere, T., Acta. Neuropathol. 97 (1999) 221-230). However, a high affinity, highly selective monoclonal antibody has not been available for this phosphoepitope until now. Such an antibody must be capable of recognising tau that is phosphorylated at Ser422 against a high background of non-phosphorylated tau and of tau phosphorylated at other pathogenic and non-pathogenic epitopes. The high degree of selectivity is crucial for allowing the clear distinction of pathological from non-pathological events in AD.

A number of publications point to the importance of the single phosphoepitope, phospho-Ser422, in the development of tau pathology.

In addition, mass spectrometric analysis of tau derived from NFTs or from normal brain showed that this phosphoepitope is unique to PHF-tau (Hasegawa, M., J. Biol. Chem. 267 (1992) 17047-17054, Cripps, D., J. Biol. Chem. 281 (2006) 10825-10838).

There are many phosphoepitopes in PHF-tau (paired helical filament-tau) and several monoclonal antibodies recognizing one or more of these phosphoepitopes are known e.g. TG3 (pThr231), PHF-1 (pSer396/pSer404), 12E8 (pS262 /pS356), AT8 (pSer202/pT205), AT100 (pSer212/pThr214), AT180 (pThr231) and AT270 (p181) (Seubert, P., J. Biol.Chem. 270 (1995) 18917-18922, Greenberg, S., J. Biol. Chem. 267 (1992) 564-569, Jicha, G., J.Neurochem. 69 (1997) 2087-2095, Mercken, M., Acta. Neuropathol. 84 (1992) 265-272).

Some of these antibodies, for instance 12E8, AT180 and AT270, also recognize tau from healthy brains and are not truely disease-state specific. All of these antibodies (except 12E8) were raised using purified PHF-tau as an immunogen and consequently the antigenic epitope recognized by these antibodies is complex, comprising either double/multiple phosphorylations or a combination of conformational and phosphoepitope. These antibodies are therefore of limited use in analyzing the contribution of individual phosphoepitopes to the development of tau pathology.

Although many polyclonal antibodies have been raised against individual phosphoepitopes, many of these show some degree of cross-reactivity with normal tau. One monoclonal antibody, AP422, has previously been described which was raised against a tau-derived phosphopeptide containing phospho-Ser422. However, this antibody shows weak cross-reactivity with normal tau (Hasegawa, M., FEBS Letters 384 (1996) 25-30).

In order to assess the contribution of this phosphoepitope to tau pathology, and to develop immunoassays for measuring levels of this phosphotau variant in brain tissues derived from AD patients, a high affinity, highly selective monoclonal antibody showing no cross-reactivity with normal tau is required.

The present invention provides an antibody which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu. Furthermore, the present invention provides an antibody which binds to tau comprising phospho-Ser422, and does not bind to tau comprising unphosphorylated Ser422. Furthermore, the present invention provides a method for detecting tau comprising phospho-Ser422. Furthermore, the present invention provides use of the antibody for detecting tau comprising phospho Ser-422. Furthermore, the present invention provides a kit for detecting tau comprising phospho-Ser422. Furthermore, the present invention provides a cell which produces the antibody.

More specifically, the present invention provides the following (1) to (24).
(1) An antibody or fragment thereof which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu.
(2) The antibody of (1), wherein A1 is Asp, A2 is Met, A3 is Val, A4 is Asp and A5 is Asp.
(3) The antibody of (1), wherein A1 is Asn, A2 is Leu, A3 is Leu, A4 is Glu and A5 is Glu.
(4) The antibody of (1) or (2), wherein the antibody binds to tau comprising phospho-Ser422 and does not bind to tau comprising unphosphorylated Ser422.
(5) The antibody of (1) to (4), wherein the antibody binds to MAP2 comprising phospho-Ser1808 and does not bind to MAP2 comprising unphosphorylated Ser1808.
(6) The antibody of (2), wherein its Kd for the peptide consisting of Ser-Ile-Asp-Met-Val-Asp-Ser(PO₃H₂)-Pro-Gln-Leu-Ala-Thr-Leu-Ala-Asp is lower than 100nM.
(7) The antibody of (1) to (6), wherein the antibody is monoclonal antibody.
(8) The antibody of (1) to (7), wherein the antibody is produced by the hybridoma DSM ACC2762 or DSM ACC2763.
(9) A method for diagnostic of neurological disorder, which method comprises
   (a) contacting a sample with the antibody of (1) to (8); and
   (b) detecting a complex formed between the antibody and tau or MAP2.
(10) The method of (9), wherein neurological disorder is Alzheimer's disease.
(11) A method for detecting tau comprising phospho-Ser422, which method comprises
   (a) contacting a sample with the antibody of (1) to (8); and
   (b) detecting a complex formed between the antibody and tau.
(12) A method for detecting MAP2 comprising phospho-Ser1808, which method comprises
   (a) contacting a sample with the antibody of (1) to (8); and
   (b) detecting a complex formed between the antibody and MAP2.
(13) The method of (11) or (12), wherein the complex is detected by western blotting, immunohistochemistry or ELISA.
(14) Use of the antibody of (1) to (8) for diagnosis of neurological disorders.
(15) Use of (14), wherein neurological disorder is Alzheimer's disease.
(16) Use of the antibody of (1) to (8) for detecting tau comprising phospho-Ser422.
(17) Use of the antibody of (1) to (8) for detecting MAP2 comprising phospho-Ser1808.
(18) A kit for diagnosis of neurological disorders, which comprises the antibody of (1) to (8).
(19) A kit of (18), wherein neurological disorder is Alzheimer's disease.
(20) A kit for detecting tau comprising phosho-Ser422, which comprises the antibody of (1) to (8).
(21) A kit for detecting MAP2 comprising phospho-Ser1808, which comprises the antibody of (1) to (8).
(22) A cell which produces the antibody of (1) to (8).
(23) The cell of (22), wherein the cell is hybridoma.
(24) The cell of (23), wherein the hybridoma is DSM ACC2762 or DSM ACC2763.

The present invention provides an antibody which binds to an epitope consisting of Ser-Ile-A1-A2-A3- A4-Ser(PO₃H₂)-Pro-Gln-Leu-Ala-Thr-Leu-Ala-A5 (SEQ ID NO:9), and does not bind to an epitope consisting of Ser-Ile-A1-A2-A3- A4-Ser-Pro-Gln-Leu-Ala-Thr-Leu-Ala-A5 (SEQ ID NO:8), wherein A1 is Asp or Asn, A2 is Met or Leu, A3 is Val or Leu, A4 is Asp or Glu and A5 is Asp or Glu. In the present invention, "Ser(PO₃H₂)" means phosphorylated serine.

In one embodiment, the antibody of the present invention binds to an epitope consisting of Ser-Ile-Asp-Met-Val-Asp-Ser(PO₃H₂)-Pro-Gln-Leu-Ala-Thr-Leu-Ala-Asp (SEQ ID NO:6), and does not bind to an epitope consisting of Ser-Ile-Asp-Met-Val-Asp-Ser-Pro-Gln-Leu-Ala-Thr-Leu-Ala-Asp (SEQ ID NO: 4).

In the preferred embodiment, the present invention provides the antibody which binds to phosphorylated tau comprising phospho-Ser422, and does not bind to tau comprising unphosphorylated Ser422.

There are several splicing isoforms of tau, such as, Fetal-tau, Tau-A, Tau-B, Tau-C, Tau D, Tau-E, Tau-F (Swiss-Prot; Entry name:TAU_HUMAN, Primary accession number:P10636). In the present invention, preferably, tau is a polypeptide having the sequence of SEQ ID NO:1. The term "tau" also contains splicing isoforms, variant, derivatives, homologues or fragments of the polypeptide having the sequence of SEQ ID NO:1. In the case tau is splicing isoform, variant, derivative, homologue or fragment of the polypeptide having the sequence of SEQ ID NO:1, it is preferable that tau comprises the sequence of Ser-Ile-Asp-Met-Val-Asp-Ser-Pro-Gln-Leu-Ala-Thr-Leu-Ala-Asp.

The term "phosphorylated tau" refers to tau in which at least one amino acid is phosphorylated.

The term "phospho-Ser422" refers to a phosphorylated Serine at position 422 as defined by the position in the amino acid sequence of SEQ ID NO:1. The term "phospho-Ser422" also contains a phosphorylated Serine in splicing isoform, derivative, variant, homologue or fragment, which is corresponding to the phosphorylated Serine at position 422 of the polypeptide having the sequence of SEQ ID NO:1.

It is preferable that the antibody of the present invention has high binding affinity to tau comprising phospho-Ser422. The term "high binding affinity", as the term is used herein, means dissociation constant K_{d} of an antibody for tau comprising phospho-Ser422 is lower than 100nM. K_{d} of an antibody can be determined by methods known to those skilled in the art. For example, K_{d} can be determined using surface plasmon resonance with Biacore under the condition described in the example 4. To use the peptide consisting of Ser-Ile-Asp-Met-Val-Asp-Ser(PO₃H₂)-Pro-Gln-Leu-Ala-Thr-Leu-Ala-Asp for determining K_{d} is preferable.

In one embodiment, the antibody of the present invention binds to an epitope consisting of Ser-Ile-Asn-Leu-Leu-Glu-Ser(PO₃H₂)-Pro-Gln-Leu-Ala-Thr-Leu-Ala-Glu (SEQ ID NO:7), and does not bind to an epitope consisting of Ser-Ile-Asn-Leu-Leu-Glu-Ser-Pro-Gln-Leu-Ala-Thr-Leu-Ala-Glu (SEQ ID NO:5).

In a preferred embodiment, the antibody of the present invention binds to MAP2 (microtubule-associated protein 2) comprising phospho-Ser1808, and does not bind to MAP2 comprising unphosphorylated Ser1808.

There are a few isoforms of MAP2, such as isoform1, isoform2 or isoform3 (Swiss-Prot; Entry name:MAP2_HUMAN, Primary accession number:P11137). In the present invention, preferably, MAP2 is a polypeptide having the sequence of SEQ ID NO:2. The term "MAP2" also contains splicing isoform, variant, derivative, homologue or fragment of the polypeptide having the sequence of SEQ ID NO:2. In the case MAP2 is splicing isoform, variant, derivative, homologue or fragment of the polypeptide having the sequence of SEQ ID NO:2, it is preferable that MAP2 has the sequence of Ser-Ile-Asn-Leu-Leu-Glu-Ser-Pro-Gln-Leu-Ala-Thr-Leu-Ala-Glu.

The term "phosphorylated MAP2" refers to MAP2 in which at least one amino acid is phosphorylated.

The term "phospho-Ser1808" refers to a phosphorylated Serine at position 1808 as defined by the position in the amino acid sequence of SEQ ID NO:2. The term "phospho-Ser1808" also contains a phosphorylated Serine in splicing isoform, derivative, variant, homologue or fragment, which is corresponding to the phosphorylated Serine at position 1808 of the polypeptide having the sequence of SEQ ID NO:2.

Whether an antibody binds to the epitope or not can be determined by well known methods, such as western blot, enzyme immunoassay or surface plasma resonance. A polypeptide consisting of the sequence of the epitope, a whole protein comprising the sequence of the epitope or a fragment of the protein can be used for detecting the binding.

The antibody of the present invention may be polyclonal or monoclonal antibody. In a preferable embodiment, the antibody of the present invention is a monoclonal antibody.

The term "monoclonal antibody" refers to an antibody obtained from a group of substantially homogeneous antibodies, i.e. , antibody group wherein the antibodies constituting the group are homogeneous except for naturally occurring mutants that exist in a small amount.

In a preferred embodiment, the antibody of the present invention is the antibody produced by the hybridoma MAK<pTAU>M.2.5.2 or MAK<pTAU>M.2.20.4. The hybridoma MAK<pTAU>M.2.5.2 was deposited at DSMZ (Deutsche Sammlung von Mikroorganismen und Zellkulturen GmBH, Mascheroderweg 1b, D-38124 Braunschweig, Germany) on March 15, 2006 under No. DSM ACC2762. The hybridoma MAK<pTAU>M.2.20.4 was deposited at DSMZ (Deutsche Sammlung von Mikroorganismen und Zellkulturen GmBH, Mascheroderweg 1b, D-38124 Braunschweig, Germany) on March 15, 2006 under No. DSM ACC2763.

The antibody produced by the hybridoma DSM ACC2762 (MAK<pTAU>M.2.5.2) or DSM ACC2763 (MAK<pTAU>M.2.20.4) has high affinity and high selectivity with tau comprising phospho-Ser422, and does not have cross-reactivity with tau comprising unphosphorylated Ser422. Additionally, the antibody produced by the hybridoma binds to MAP2 comprising phospho-Ser1808, and does not bind to MAP2 comprising unphosphorylated Ser1808. These antibodies can be used for detecting tau comprising phospho-Ser422 or MAP2 comprising phospho-Ser1808. The antibody produced by the hybridoma DSM ACC2762 or DSM ACC2763 is suitable for diagnosis of neurological diseases such as Alzheimer's disease. The antibody produced by the hybridoma DSM ACC2762 or DSM ACC2763 is also suitable for detecting tau comprising phospho-Ser422 or MAP2 comprising phospho-Ser1808.

The antibody of the present invention can be obtained as a polyclonal antibody or a monoclonal antibody, using known techniques. For example, the monoclonal antibody can be produced by the hybridoma method (Kohler G. and Milstein C., Nature 256 (1975) 495-497) or the recombinant method (U.S. patent No. 4,816,567). The monoclonal antibody can be also isolated from a phage antibody library (Clackson T., Nature 352(1991) 624-628).

Hybridoma producing the antibody can be prepared essentially using known techniques. For example, following methods can be used: An animal is immunized by a conventional immunization method using a sensitizing antigen to obtain an immune cell, which is then fused to a known parent cell by a conventional cell fusion method. Fused cells are screened for monoclonal antibody-generating cells by a conventional screening method. More specifically, the monoclonal antibody can be obtained as follows.

First, a fragment which has the sequence of Ser-Ile-A1-A2-A3- A4-Ser-Pro-Gln-Leu-Ala-Thr-Leu-Ala-A5 (A1 is Asp or Asn, A2 is Met or Leu, A3 is Val or Leu, A4 is Asp or Glu and A5 is Asp or Glu), for use as a sensitized antigen is prepared. The fragment can be prepared by chemical synthesis or culturing host cell comprising a gene coding the fragment. Then, the fragment is phosphorylated by kinase, for example ERK2 kinase. A whole protein comprising the fragment may also be used as a sensitized antigen. In the present invention, preferable sensitized antigen is the phosphorylated fragment of tau, which has the sequence of Ser-Ile-Asp-Met-Val-Asp-Ser(PO₃H₂)-Pro-Gln-Leu-Ala-Thr-Leu-Ala-Asp.

There is no limitation as to the type of mammalian species to be immunized with the sensitized antigen. However, a mammal is preferably selected based on its compatibility with the parental cell to be used in cell fusion. Generally, rodents such as for example mice, rats, hamsters or rabbits, and monkeys can be used.

For immunization of animals with a sensitizing antigen, known methods may be employed. Animals can be immunized with a sensitized antigen by well known methods such as a routine method of injecting a sensitized antigen into a mammal intraperitoneally or subcutaneously. Specifically, the sensitized antigen is diluted appropriately with phosphate-buffered saline (PBS), physiological saline and such, and then suspended. An adequate amount of a conventional adjuvant, for example, Freund's complete adjuvant, is mixed with the suspension, as necessary. An emulsion is then prepared for administering to a mammal several times over a 4- to 21-day interval. An appropriate carrier may be used for the sensitized antigen in immunization.

A mammal is immunized as described above. After a titer increase of target antibody in the serum is confirmed, immunocytes are collected from the mammal and then subjected to cell fusion. Spleen cells are the preferred immunocytes.

Mammalian myeloma cells are used as the parental cells to be fused with the above immunocytes. Preferable myeloma cells to be used include various known cell lines, for example, P3(P3x63Ag8.653) (Kearney JF, et al., J. Immnol. 123, 1548-1550 (1979)), P3x63Ag8U.1 (Yelton DE, et al., Current Topics in Microbiology and Immunology 81, 1-7 (1978)), NS-1 (Kohler, G. and Milstein, C. Eur. J. Immunol. 6, 511-519 (1976)), MPC-11 (Margulies, D. H. et al., Cell 8, 405-415 (1976)), SP2/0 (Shulman, M. et al., Nature 276, 269-270 (1978)), FO (deSt. Groth, S. F. et al., J. Immunol. Methods 35, 1-21 (1980) ), S194 (Trowbridge, I. S., J. Exp. Med. 148, 313-323 (1978)), and R210 (Galfre, G. et al., Nature 277, 131-133 (1979)).

Cell fusions between the immunocytes and the myeloma cells as described above can be essentially carried out using methods known to a person skilled in the art, for example, a method by Kohler and Milstein (Kohler, G. and Milstein, C., Methods Enzymol. 73: 3-46 (1981)).

More specifically, the above-described cell fusions may be carried out, for example, in a conventional culture medium in the presence of a cell fusion- promoting agent. The fusion-promoting agents include but are not limited to polyethylene glycol (PEG) and Sendai virus (HVJ). If required, an auxiliary substance such as dimethyl sulfoxide may also be added to improve fusion efficiency.

The ratio of immunocytes to myeloma cells may be determined at one's own discretion, preferably, one myeloma cell for every one to ten immunocytes. Culture media to be used for the above cell fusions include, for example, media that are suitable for the growth of the above myeloma cell lines, such as RPMI 1640 media and MEM media, and other conventional culture media used for this type of cell culture. In addition, serum supplements such as fetal calf serum (FCS) may also be used in combination.

Cell fusion is carried out as follows. As described above, predetermined amounts of immunocytes and myeloma cells are mixed well in the culture medium. PEG solution (for example, mean molecular weight of about 1,000- 6,000) pre-heated to 37°C is added to the cell suspension typically at a concentration of 30% to 60% (w/v), and mixed to produce fused cells (hybridomas). Then, an appropriate culture medium is successively added to the mixture, and the sample is centrifuged to remove supernatant. This treatment is repeated several times to remove the unwanted cell fusion-promoting agent and others that are unfavorable to hybridoma growth.

Screening of the resulting hybridomas can be carried out by culturing them in a conventional selective medium, for example, hypoxanthine, aminopterin, and thymidine (HAT) medium. Culturing in the above-descried HAT medium is continued for a period long enough (typically, for several days to several weeks) to kill cells (non-fused cells) other than the desired hybridomas. Then, hybridomas are screened for single-cell clones capable of producing the target antibody by conventional limiting dilution methods.

In addition to the method for preparing the above-described hybridomas by immunizing non-human animals with antigens, human antibodies can also be obtained by sensitizing human lymphocytes with a sensitized antigen in vitro; and fusing the sensitized lymphocytes with human myeloma cells capable of dividing permanently (see, for example, Japanese Patent Application No. (JP-B) Hei 1-59878).

Alternatively, it is possible to obtain human antibodies from immortalized cells producing the antibodies. In this method, the cells producing the antibodies are prepared by administering a sensitized antigen to transgenic animals comprising a repertoire of the entire human antibody genes (see for example WO 94/25585, WO 93/12227, WO 92/03918, and WO 94/02602).

The monoclonal antibody-producing hybridomas thus prepared can be passaged in a conventional culture medium, and stored in liquid nitrogen over long periods of time.

Monoclonal antibodies can be prepared from the above-described hybridomas by, for example, a routine procedure of culturing the hybridomas and obtaining antibodies from the culture supernatants.

Once monoclonal antibody is obtained from a hybridoma, recombinant antibody can also be prepared by conventional methods.

The antibody of the present invention may be a labelled antibody. For the labelling of the antibody, known labels such as radioactive substance (for example, ³²P, ¹²⁵I, ³H, ¹³¹I, ¹⁴C), fluorochrome (for example fluorescein, rhodamine, umbeliferone), enzyme (for example luciferases, oxidase, allakine phosphatase, β-galactosidase, β-glucosidase, lysozyme, glucoamylase), coenzyme (for example biotin) or chemiluminescence substance can be used. The labeling of the antibody can be done by well known methods, such as for example glutaraldehyde method, maleimide method, pyridyl disulfide method or periodic acid method.

The antibody of the present invention may also be fragment of the antibody as long as the fragment can bind to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5 (A1 is Asp or Asn, A2 is Met or Leu, A3 is Val or Leu, A4 is Asp or Glu and A5 is Asp or Glu). The fragment of antibody may be, but not limited, Fab, Fab', Fv, F(ab')2, scFv, sc(Fv)2 or diabody.

Preferably, the antibody of the present invention forms an immunological complex with tau or fragment thereof comprising a phosphorylated Ser at position 422 as defined by the position of the amino acid in SEQ ID NO:1. More preferably, said tau or fragment thereof is isolated from autopsy-derived brain tissue of AD patients or patients having any other neurological disorders with NFT present. Preferably, the antibody of the present invention does *not* form such an immunological complex with autopsy-derived brain material from patients having died or suffering from other diseases without the occurrence of NFTs in the brain.

The antibody of the present invention can be used for diagnosis of a neurological disorder such as Alzheimer's disease by detecting phosphorylated polypeptide. The antibody of the present invention can be also used for detection of phosphorylated polypeptide comprising the sequence of Ser-Ile-A1-A2-A3- A4-Ser(PO₃H₂)-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu, such as phosphorylated tau comprising phospho-Ser422 or phosphorylated MAP2 comprising phosho-Ser1808.

Moreover, the present invention provides a cell which produces the antibody of the present invention. The cell of the present invention may be a hybridoma or a recombinant cell.

The hybridoma which produces the antibody of the present invention can be obtained by conventional methods described above. In a preferable embodiment, hybridoma of the present invention is hybridoma DSM ACC2762 or DSM ACC2763. The hybridoma DSM ACC2762 was deposited at DSMZ (Deutsche Sammlung von Mikroorganismen und Zellkulturen GmBH, Mascheroderweg 1b, D-38124 Braunschweig, Germany) on March 15, 2006 under No. DSM ACC2762. The hybridoma DSM ACC2763 was deposited at DSMZ (Deutsche Sammlung von Mikroorganismen und Zellkulturen GmBH, Mascheroderweg 1b, D-38124 Braunschweig, Germany) on March 15, 2006 under No. DSM ACC2763.

A recombinant cell of the invention can be obtained by well known methods. Specifically, such recombinant cell can be obtained as follows.

An mRNA encoding the variable (V) region of the antibody which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu, is isolated from hybridomas producing such an antibody.

For mRNA isolation, total RNAs are first prepared by conventional methods such as guanidine ultracentrifugation methods (Chirgwin, J. M. et al., Biochemistry 18, 5294-5299 (1979)), or acid guanidinium thiocyanate-phenol- chloroform (AGPC) methods (Chomczynski, P. et al., Anal. Biochem. 162, 156-159 (1987)), and then the target mRNA is prepared using an mRNA Purification Kit (Pharmacia) and such. Alternatively, the mRNA can be directly prepared using the QuickPrep mRNA Purification Kit (Pharmacia).

A cDNA of the antibody V region is synthesized from the resulting mRNA using reverse transcriptase. cDNA synthesis is carried out using the AMV Reverse Transcriptase First-strand cDNA Synthesis Kit (Seikagaku Co.), or such. Alternatively, cDNA can be synthesized and amplified by the 5'-RACE method (Frohman, M. A. et al., Proc. Natl. Acad. Sci. USA 85, 8998-9002 (1988); Belyavsky, A. et al., Nucleic Acids Res. 17, 2919-2932 (1989)) using the 5'- Ampli FINDER RACE Kit (Clontech) and PCR.

Target DNA fragments are purified from the obtained PCR products and then ligated with vector DNAs to prepare recombinant vectors. The vectors may be introduced into E. coli and such, and colonies are selected for preparing the recombinant vector of interest. The target DNA nucleotide sequence is then confirmed by conventional methods such as the dideoxynucleotide chain termination method.

Once a DNA encoding the V region of target antibody is obtained, the DNA is inserted into an expression vector which comprises a DNA encoding the constant region (C region) of a desired antibody.

The method for obtaining the cells which produce the antibody typically comprises the steps of: inserting the antibody gene into an expression vector, so that the gene is expressed under the regulation of expression regulatory regions, such as enhancer and promotor; and transforming host cells with the resulting vectors to express the antibody. Polynucleotide encoding H chain and L chain, respectively, can be inserted into separate expression vectors and co- transfected into a host cell. Alternatively, polynucleotides encoding both H chain and L chain can be inserted into a single expression vector and transfected into a host cell (see for example WO 94/11523). The host cells are not limited as long as the cells can produce the antibody. For example, the cells may be CHO, COS, NIH3T3, myeloma, BHK, Hela, Vero, amphibian cells, insect cells, plants cells or bacterial cells such as *E. coli.*

Furthermore, the present invention provides a method for diagnosis of neurological disorder. The method for diagnosis of neurological disorder comprises the following steps:(a) contacting a sample with the antibody of the present invention, and (b) detecting the complex formed between the antibody and tau or Map2.

The present invention further provides a method for detecting tau comprising phospho-Ser422. The method for detecting tau comprising phospho-Ser422 comprises
(a) contacting a sample with the antibody of the present invention; and (b) detecting a complex formed between the antibody and tau.

The present invention further provides a method for detecting MAP2 comprising phospho-Ser1808. The method for detecting MAP2 comprising phospho-Ser1808 comprises (a) contacting a sample with the antibody of the present invention; and (b) detecting a complex formed between the antibody and MAP2.

The term "neurological disorder", as used herein, refers to disease which are related to tau comprising phospho-Ser422 or MAP2 comprising phospho-Ser1808, such as diseases caused by tau comprising phospho-Ser422 or MAP2 comprising phospho-Ser1808, or diseases of which tau comprising phospho-Ser422 or MAP2 comprising phospho-Ser1808 is detected from a patient. The term "neurological disorder" includes, but is not limited to, Alzheimer's disease, Down's Syndrome, Pick's Disease, progressive supranuclear palsy and corticobasal degeneration.

Although a sample used in the methods of the present invention are not limited as long as there is a possibility the sample comprises tau or MAP2, a biological sample obtained from human is preferred. A biological sample includes, but is not limited to, cerebrospinal fluid, serum, blood, tissue (for example, brain tissue) and cell (for example, brain cell).

A contact of a sample with the antibody of the present invention can be achieved under conditions which allow the formation of a complex formed between the antibody and tau or a complex formed between the antibody and MAP2. Such conditions are well known to a person skilled in the art.

The detection of a complex formed between the antibody and tau or a complex formed between the antibody and MAP2 can be carried out by conventional manner, such as western blot, enzyme immunoassay such as enzyme-linked immunosorbant assay(ELISA), radioimmunoassay, fluorescent immunoassay, luminescent immunoassay, immunoprecipitation, immunostaining, immunodiffusion and surface plasma resonance biosensor (such as BIAcore).

The method of the present invention may be carried out *in vitro* or *in vivo,* however, *in vitro* method is preferable.

The present invention also provides use of the antibody of the present invention for diagnosis of neurological disorders, such as Alzheimer's disease. The present invention also provides use of the antibody of the present invention for detecting a phosphorylated polypeptide such as tau comprising phospho-Ser422 or MAP2 comprising phospho-Ser1808.

The antibody of the present invention may be used *in vivo* or *in vitro.* In the preferred embodiment, the antibody of the present invention is used *in vitro.*

The present invention provides a kit comprising the antibody which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu. This kit can be used, for example, for diagnosis of neurological disorders, detecting tau comprising phospho-Ser422 or detecting MAP2 comprising phospho-Ser1808.

The kit may comprise a carrier, such as insoluble polysaccharide(for example, agarose or cellulose), synthetic resin(for example, polystyrene resin, silicon resin, polyacrylamide resin, polycarbonate resin or nylon resin) or insoluble support(for example, glass). The antibody may be immobilized to the carrier.

The kit may comprise a reagent, such as reaction liquid, blocking solution, or other reagent used in immunoassay.

Having now generally described this invention, the same will become better understood by reference to the specific examples, which are included herein for purpose of illustration only and are not intended to be limiting unless otherwise specified, in connection with the following figures.

### Figures

**Figure 1** shows specificity of the monoclonal antibodies. Specificity of the monoclonal antibodies was tested by western blot analysis of various tau species with 441aa. A: Pan α-tau (control), antibodies 2.5.2 and 2.20.4; B: monoclonal antibody AP422
   Lane1 : tau comprising unphosphorylated Ser at position 422
   Lane2 : phosphorylated tau comprising phosphorylated Ser at position 422
   Lane3 : tau comprising Ser→Ala mutation at position 422
   Lane4 : phosphorylated tau comprising Ser→Ala mutation at position 422
**Figure 2** shows the result of western blot analysis of soluble- and insoluble extracts from Braak-staged AD brain tissue. Several tau bands are visible due to the fact that the human brain contains multiple splicing isoforms of tau. Note that neither Braak stage II/control brain, nor the soluble fraction, contain tau phosphorylated at Ser422. M = lane containing marker proteins (Novex); I= insoluble fraction; S= soluble fraction; II, IV, VI= Disease severity (Braak stage)
**Figure 3** shows the result of Immunohistochemical analysis of AD brain tissue (Braak stage VI). NFT = neurofibrillary tangle; NT = neuropil thread; DN = dystrophic neuritis (surrounding amyloid plaque). Antibody used was 2.5.2. Control brain showed no staining.
**Figure 4** shows the result of western blot analysis of okadaic acid-treated LAN-5 cells (OA) using antibody 2.5.2. The antibody reacted with three proteins which were all phosphoproteins, as indicated by the lack of reactivity with vehicle/DMSO extract or with extract from cells treated with the pan-kinase inhibitor K252a. The individual molecular weights were consistent with the largest protein being MAP 2a/b (phosphorylated at S1808) and the two smaller proteins being two different isoforms of tau (phosphorylated at S422). Lane 1 = vehicle; lane 2 = OA, lane 3 = OA + K252a.
**Figure 5** shows the result of ELISA for determination of tau/pser422 levels in okadaic acid-treated LAN-5 cells. The amount of tau/pSer422 was calculated on the basis of 1 x 10⁶ cells.

### EXAMPLES

The following examples serve merely to illustrate certain aspects of the present invention and should not be viewed as limiting the present invention in scope.

### Example 1: Generation of monoclonal antibodies

### A. Immunization of mice

Mice were immunized with a phosphopeptide comprising the following amino acid sequence
Cys-Ser-Ile-Asp-Met-Val-Asp-Ser(PO₃H₂)-Pro-Gln-Leu-Ala-Thr-Leu-Ala-Asp
which corresponds to amino acids 415-430 of the longest isoform of human tau (peptide synthesized by NeoMPS, Strasbourg). The naturally occurring Asp 415 was replaced by Cys to allow directed coupling via the thiol to KLH.

10-12-week old female Balb/c (Jackson Laboratory, stock#001026) and NMRI mice (Jackson Laboratory, stock#003076) were injected intraperitoneally with 100 µg of phosphopeptide in Complete Freund's Adjuvant. The mice received three further interperitoneal injections of 100 µg peptide in Freund's Incomplete Adjuvant at monthly intervals. Final immunizations were made at 3, 2 and 1 day before spleen removal by intravenous injection of 50 µg peptide in PBS.

### B. Fusion and Cloning

Fusion of the spleen cells from immunized mice was carried out according to Galfré and Milstein, Methods in Enzymology, 73 (1981) 3-46. Thus 1 x 10⁸ spleen cells from each immunized mouse were mixed with 2 x 10⁷ myeloma cells (P3X63-Ag8-653, ATCC CRL1580) and centrifuged at 300 x *g* for 10 min at RT. The cells were then washed once with RPMI-1640 medium (without FCS) and centrifuged once more at 300 x *g* for 10 min in a conical tube. The cells were loosened by tapping the tube and warmed to 37°C in a pre-heated waterbath . Within the space of 1 minute, 1 ml of PEG was added (polyethylenglycol with molecular weight 1500, Roche Diagnostics). Next, 5 ml of RPMI-1640 medium (without FCS) was added dropwise with gentle shaking and finally the volume was adjusted to 50 ml by addition of RPMI-1640 medium containing 10% FCS. The cell suspension was then centrifuged at 300 x g for 10 min. The cell pellet was resuspended in RPMI-1640 medium containing 10% FCS and seeded into hypoxanthine-azaserine selection medium (100 mM hypoxanthine [Sigma], 1 µg/ml azaserine [Sigma] in RPMI-1640 + 10% FCS). The medium was supplemented with 50U/ml interleukin-6 (Roche Applied Science).

After 10 days the primary cultures were tested for specific antibody production (see Example 2). Primary cultures which showed a positive reaction with phosphopeptide and no reaction with unphosphorylated peptide were cloned by fluorescent-activated cell sorting in 96-well cell culture plates. The medium used here was RPMI-1640 medium supplemented with 10%FCS and 25 U/ml interleukin-6.

Two of the hybridoma cell lines/clones obtained in this way were deposited at DSMZ (Deutsche Sammlung von Mikroorganismen und Zellkulturen GmbH, Mascheroder Weg 1b, D-38124 Braunschweig).

Table 1 shows the details of the hybridoma.

**Table 1**

| **Clone** | **Deposited at DSMZ on 15.03.2006 under the following number** | **IgG Subclass** |
|---|---|---|
| 2.5.2 | DSM ACC2762 | Ig G2a; κ-light chain |
| 2.20.4 | DSM ACC2763 | Ig G2a; κ-light chain |

### C. Antibody purification from cell culture supernatant

Hybridoma cells thus obtained were seeded at a density of 1 x 10⁵ cells/ml in RPMI-1640 medium supplemented with 10% FCS and 25U/ml interleukin-6 and grown to a cell density of about 3 x 10⁵/ml. The cells were then diluted to 1 x 10⁶/ml in a final volume of 250 ml and allowed to grow to maximal cell density. The cells were then removed by centrifugation. The hybridoma culture supernatants typically contained ca. 40-50 µg/ml antibody.

Each antibody was purified as follows. Cell-free hybridoma culture supernatant (250-300 ml) was loaded on a 25 ml MEP HyperCel column (Pall Biosciences) which was equilibrated with 50 mM TrisCl pH 8.0. After washing with equilibration buffer the antibody was eluted with 30 mM sodium citrate /100 mM NaCl pH 4.1. Antibody containing fractions were pooled and then dialysed (Spectra/Por 6-8000) overnight at 4°C against 5 1 of SourceQ buffer A. The dialysed MEP pool was loaded on a 10 ml Source 15Q column (GE Healthcare) which was equilibrated with 10 mM TrisCl pH 8.0 (buffer A). After washing with buffer A the antibody was eluted with a gradient from 0 - 25 % buffer B in 10 column volumes. Buffer B contained 10 mM TrisCl /1 M NaCl pH 8.0. The antibody eluted at about 200 mM NaCl. The purity of the individual fractions was checked by SDS-PAGE and the purest fractions pooled. The yield of antibody thus purified was ca. 10 mg/250 ml hybridoma culture supernatant.

### Example 2: Screening for anti-Tau/pSer422 specific antibodies

### A. Determination of specificity of antibodies for phosphopeptide (tau 416-430/pSer422)

In order to measure the specificity of the antibodies in the cell culture supernatants, streptavidin-coated microtiter plates (MicroCoat, Bernried, DE) were coated with 0.1 µg/ml biotinylated phosphopeptide (tau 416-430/pSer422) in PBS, 0.5% Byco C (100 µl/well, 1 h incubation at R.T. with shaking). The plates were then washed 3 times with wash buffer (0.9 % NaCl / 0.05% Tween 20). Next, 100 µl of antibody-containing culture supernatant was added in each well and the plates incubated for 1 h at room temperature (R.T.) with shaking. The plates were then washed 3 times with wash buffer. In order to detect bound antibody, 100 µl/well of a polyclonal anti-mouse antibody /peroxidase conjugate (Dianova) for 1 h at R.T.. The plates were subsequently washed again. Finally, 100 µl/well of ABTS solution (Roche Diagnostics) was added and the plates incubated for 20 min at R.T. The plates were then read at 405 nm in an X Read Plus microplate reader (Tecan) .

### B. Measurement of antibody cross-reactivity with non-phosphorylated peptide (tau 416-430)

The same procedure as described above was used, except that the microtitre plates were coated with non-phosphorylated peptide (tau 416-430).

### C. Measurement of binding to free phosphopeptide (tau 416-430/pSer422)

100 µl/ well of each cell culture supernatant was pipetted into a microtitreplate that had previously been coated with anti-mouse Fcγ antibody (MicroCoat, Bernried, DE). The plate was then incubated for 1 h at R.T. with shaking and subsequently washed 3 times with wash buffer. Next, 100 µl/well of 50 nM biotinylated phosphopeptide (tau 416-430/pSer422) was added and the plate incubated for 1 h at R.T. with shaking. The plate was washed three times and then incubated with 100 µl/well of 50U/ml streptavidin/peroxidae conjugate (Dianova) for 1h at R.T. The plate was washed once again and then developed with 100/µl well ABTS solution (Roche Applied Science) for 20 min at R.T. The plate was then read at 405 nm in a X Read Plus microplate reader.

### D. Measurement of antibody binding to free non-phosphorylated peptide (tau 416-430)

The same procedure as described above was used, except that the microtitre plates were coated with non-phosphorylated peptide (tau 416-430).

Using the methods described above, both of the deposited antibodies showed good binding to immobilized and free phosphopeptide (tau 416-430/pSer422). No cross-reactivity was observed with non-phosphorylated peptide (tau 416-430).

### Example 3: Determination of antibody specificity towards full-length phosphorylated tau protein and phosphorylated mutant tau protein (S422A) by western blot

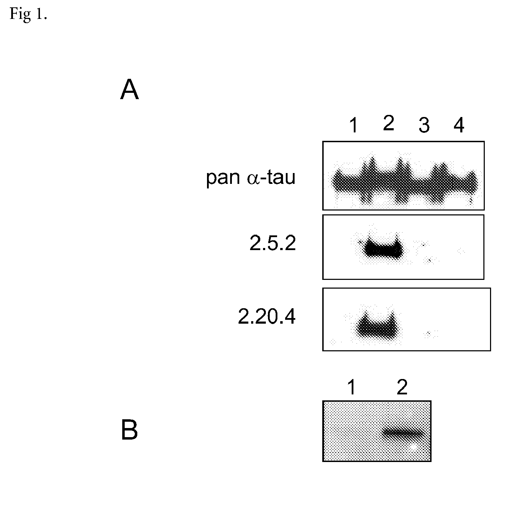

Antibodies were tested for their ability to recognise each of four different tau species, namely tau, tauS422A (Ser→Ala mutation at position 422), p-tau (phosphorylated tau) and p-tauS422A (phosphorylated tau comprising Ser→Ala mutation at position 422). Tau and tauS422A were expressed in *E. coli* and purified according to standard method. Both proteins were then phosphorylated by ERK2 kinase, which was shown to introduce a phosphogroup at S422 as well as a number of other sites in tau. SDS-PAGE gel was loaded with 150 ng of each of the four tau species; subsequent to electrophoresis the proteins were transferred to nitrocellulose by standard western blotting protocol. Blots were incubated overnight at 4°C with individual hybridoma cell culture supernatants that had been diluted two-fold with StartingBlock (Perbio). After a standard washing procedure, the blots were incubated in 2 ng/ml anti-mouse antibody /horseradish peroxidase conjugate (Perbio) for 1 hr at RT. The blots were then washed and developed with LumiLight ECL substrate (Roche Applied Science). As shown in Figure 1A, antibodies 2.5.2 and 2.20.4 (table 1) showed no cross-reactivity with non-phosphorylated tau nor with tau phosphorylated at Ser (or Thr) residues other than Ser422. The antibodies are thus highly selective for the target phosphoepitope. Figure 1B shows the specificity of the monoclonal antibody AP422. The monoclonal antibody AP422 was obtained from Prof. M. Hasegawa (University of Tokyo, Japan) and diluted 1:2000 with 1% milk in TBSt buffer. The blot was incubated overnight at 4°C. The following day it was washed and incubated for 1 hr at room temperature with anti-mouse/horseradish peroxidase (Pierce) diluted 1:5000 with 1% milk in TBSt buffer. Detection was with the Roche ECL system. The antibody AP422 showed cross-reactivity with unphosphorylated tau.

### Example 4: Determination of kₒₙ, k_{off}, K_{A} and K_{d}

The rate constants kₒₙ and k_{off} and the resulting dissociation constant K_{d} were determined using surface plama resonance (BIAcore 2000 from BIAcore AB).

NHS/EDC-activated Sensorchips (CM5, BIAcore AB) were coated with rabbit-anti mouse IgG at a concentration of 15 µg/ml in 10mM sodium acetate-acetic acid pH5.0 for 5 min at a flow rate of 20 µl/min. Cell culture supernatants were diluted to a final antibody concentration of 50 nM in 10 mM HEPES pH 7.4, 150 mM NaCl, 3.4 mM EDTA, 0.05% Polysorbate and injected at a flow rate of 10 µl/min over a period of 2 min. Phosphopeptide or non-phosphorylated peptide (0-1000 nM) in 10 mM HEPES pH 7.4, 150 mM NaCl, 3.4 mM EDTA, 0.05% Polysorbate was then injected at a flow rate of 100 µl/min for 2 min. This was followed by dissociation for 5 min in 10 mM HEPES pH 7.4, 150 mM NaCl, 3.4 mM EDTA, 0.05% Polysorbate.

kₒₙ and k_{off} were then calculated from the sensogram after double referencing using BIAcore evaluation software (version 4.1, BIAcore AB). Models were fitted gobally across the data sets using the 1:1 (Langmuir) binding interaction. The association constant Kₐ was calculated from kₒₙ / k_{off}

The values obtained are summarized below. Both antibodies have K_{d} values in the low nanomolar range and thus can be considered to bind pSer422 with high affinity. Neither of the antibodies showed any interaction with the non-phosphorylated peptide, indicating that they are highly selective for the phosphoepitope and confirming the western blot results in example 3.

Table 2 shows Kₒₙ, K_{off}, Kₐ and K_{d} of the antibody 2.5.2 and the antibody 2.20.4.

**Table 2**

| | Tau 416-430/pSer422 | | | |
|---|---|---|---|---|
| Clone | **k**ₒₙ | **k**_{off} | **K**ₐ | **K**_{d} |
| | 1/Ms | 1/s | 1/M | nM |
| 2.5.2 | 8 x 10⁵ | 8 x 10⁻³ | 9 x 10⁷ | 11 |
| 2.20.4 | 7 x 10⁵ | 1 x 10⁻² | 7 x 10⁷ | 14 |

### Example 5: Western blots of AD brain extracts

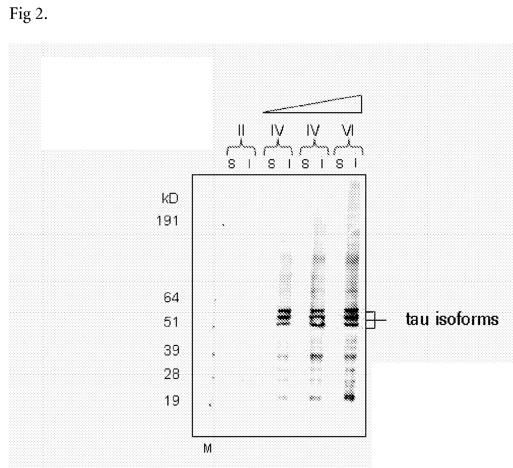

Microtome sections were prepared from Braak-staged AD brains and control brains. Ca. 50 mg wet weight tissue from each brain was dispensed into Eppendorf tubes. Each tissue was then homogenized in 10 volumes of ice-cold RAB-HS buffer using a hand-held glass-homogenizer. Homogenates were then centrifuged at 50000xg for 40 min at 4°C. The resulting pellets were resuspended in Tris-sucrose-SDS by homogenization and centrifuged at 50000xg for 40 min at RT. The resulting supernatants were retained for analysis. SDS-PAGE gel was loaded with 4.5 µl each supernatant. Western blotting was carried out as described above, except that the first antibody (2.5.2, purified as described above) was diluted to 1 µg/ml in StartingBlock. Results are shown in Figure 2. The antibody specifically detected tau isoforms phosphorylated at Ser422 in the RAB-insoluble/SDS-soluble brain extracts. This is to be expected since hyperphosphorylated tau usually occurs in this fraction of the extract. Normal tau occurs in the RAB-soluble fraction and clearly there is no cross-reaction of the antibody with this fraction in any of the samples. The intensity of staining correlates with disease severity and no staining is evident with the control brain extract.

### Example 6: Immunohistochemical analysis of AD brain sections

Cryostat sections of unfixed brain tissue from cortical human brain regions, obtained postmortem from a patient that was positively diagnosed for Alzheimer's disease, Braak stage VI. Sections were labeled by indirect immunofluorescence with anti-p-tauS422A antibody (Wheatley S. and Wang Y., Methods Cell Bio 57 (1998) 313-332). A successive two-step incubation was used to detect bound anti-p-tauS422A antibodies, which are revealed by affinity-purified goat anti-mouse (GAM) IgG (H+L) conjugated to Alexa 488 (Molecular Probes). A counterstaining against Aβ peptide was doe to reveal amyloid-β plaques.

In detail, Sections were cut at -18°C using a cryostat (Leica, CM 3050 S) at a nominal thickness of 10 µm. After adhesion to pre-cooled glass slides (Super Frost Plus, Menzel, Germany), sections were hydrated in PBS and treated with 100% acetone pre-cooled at -20°C for 2 min. Washing with PBS was done twice for two minutes. Blocking of unspecific binding sites was done by incubation in PBS with 1% bovine serum albumin (BSA) and 1% ovalbumin (OVA) and 1% normal goat serum for 20 min. Anti-p-tauS422A antibodies were used at a concentration of 10 µg/ml in PBS with 1% BSA, 1% OVA and 1% normal goat serum for 1 hour. After washing with PBS and 1% BSA, slides were incubated with affinity-purified goat anti-mouse (GAM) IgG (H+L) conjugated to Alexa 488 (Molecular Probes) at 15 ug/ml in PBS with 1% BSA for 1 hour. Slides were washed 3 x 5 minutes with PBS and 1 % BSA and counterstained with a monoclonal mouse antibody against Aβ (BAP-2, Dr. M. Brockhaus, F.Hoffmann La Roche, EP130424) at 5 µg/ml in PBS with 1% BSA for 1 hour. Slides were washed 3 x 5 minutes with PBS rinsed with water and immersed in 0.3% sudan black in 70% aqueous ethanol for 5 minutes to reduce autofluorescence of lipofuscin. After one rinse with 70% ethanol and 2 rinses with water slides were washed with 2 x 3 minutes PBS and embedded with fluorescence mounting medium (S3023 Dako). Controls included unrelated mouse IgG1 antibodies (Sigma) and the secondary antibody alone, which all gave negative results.

Images were recorded with a Zeiss Axioplan2 using a 10x/0.3 objective. Images of both recorded fluorescence channels were merged with Photoshop.

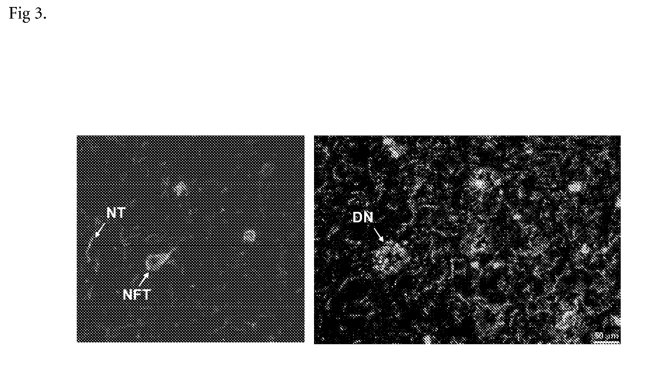

Results are shown in Figure 3. The antibody stained typical structures associated with the pathology of Alzheimer's Disease, the most prominent of these being neurofibrillary tangles . Numerous neuropil threads were also evident, as were dystrophic neurites surrounding plaques (visualised by counter staining with an amyloid-specific antibody).

### Example 7: Western blots for detecting pSer422 in tau and MAP2 in neuroblastoma cell extracts

The peptide immunogen used to generate the monoclonal antibodies is based on the amino acid sequence immediately surrounding S422 in tau, namely S416-IDMVDSPQLATLA-D430. This sequence occurs in tau towards its C-terminus. A homology search of protein sequence databases revealed that the highly homologous amino acid sequence S1802-INLLESPQLATLA-E1816 occurs towards the C-terminus of the microtubule-associated protein MAP2. LAN-5 cells treated with okadaic acid and analyzed by western blot using the antibodies described above do indeed contain a high molecular weight protein which cross-reacts with antibodies 2.5.2 and 2.20.4. The molecular weight of this protein is consistent with it being MAP2.

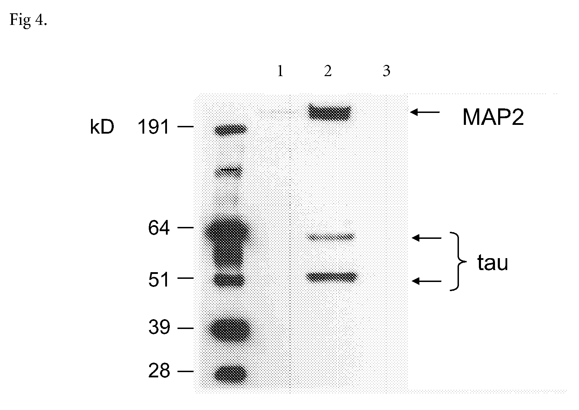

Cells (1x10⁷/well in serum-free medium) were treated with DMSO or 10µM K252a (Alexis) for 1 hr at 37°C; the medium was then supplemented with DMSO or 2 µM okadaic acid for a further 1 hr at 37°C . Medium was removed and the cells extracted into 100 µl of Cytobuster (Novagen) and 25 µl used for western blot analysis. Western blots were performed as described in example 5. The result is shown in Fig. 4. The antibody 2.5.2 showed cross-reactivity with phosphorylated MAP2.

### Example 8: ELISA assay for detecting tau/pSer422 in okadaic acid-treated neuroblastoma cells

LAN-5 neuroblastoma cells express tau endogenously. Phosphorylation of endogenous tau at Ser422 occurs when the cells are treated with okadaic acid.

LAN-5 neuroblastoma cells are cultured in medium at 37°C and plated into 96-well microtitre plates at a cell density of 2.5 x 10⁵ cells/well in 100µl of serum-free medium. After 24 h in culture the cells are then treated with 2.5 µM okadaic acid for 2h. Subsequently, 10 µl of a solution comprising 1mg/ml digitonin, 10 mM EDTA is added and the cells shaken for 30 min at 4°C. This extract is then used directly in the ELISA assay described below.

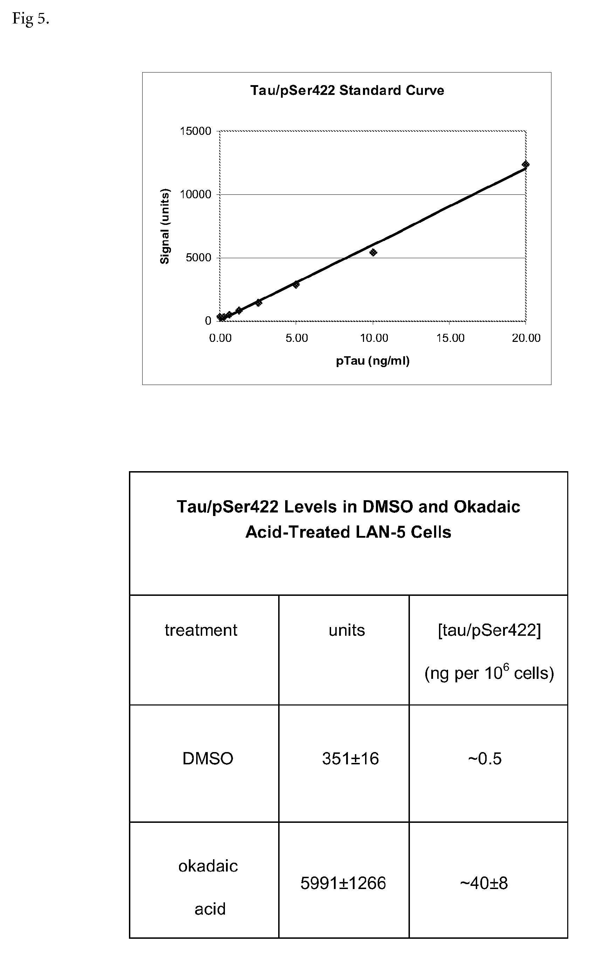

Antibody 2.5.2 was biotinylated using biotin-NHS at a ratio of 20:1 in phosphate buffered saline, pH7.2. Antibody 5A6 (Developmental Studies Hybridoma Bank, University of Iowa) was labelled with BV-TAG™ at a ratio of 8:1 according to the protocol supplied by BioVeris (BioVeris Corporation, Gaithersburg, Maryland). In a typical assay, 25 µl streptavidin-coated magnetic beads (Dynal/Invitrogen Corporation) at a dilution of 1:50 was preincubated for 30 min with 25 µl of 1 µg/ml biotin-antibody at RT. To this mixture was then added 50 µl of cell extract and 25 µl BV-tagged antibody 2.5.2 (prepared as described above). The mixture was then incubated for 3h at 4°C with shaking. All samples were prepared in 96-well microtitre plates. Subsequently 125 ul buffer was added to each sample and plate measured in BioVeris M384 analyser. Tau/pSer422 standard curve was prepared by diluting ERK2- or P38-phosphorylated tau in cell extraction buffer; calculation of tau/pSer422 level in cells assumes 1 mole phospho-Ser422 phosphate/mole tau for the phospho-tau standard. Figure 5 shows a standard curve and typical values obtained (on the basis of 1 x 10⁶ cells) before and after okadaic acid treatment.

## Claims

1. An antibody or a fragment thereof which binds to an epitope consisting of Ser-Ile-A1-A2-A3- A4-Ser(PO₃H₂)-Pro-Gln-Leu-Ala-Thr-Leu-Ala-A5, and does not bind to an epitope consisting of Ser-Ile-A1-A2-A3- A4-Ser-Pro-Gln-Leu-Ala-Thr-Leu-Ala-A5, wherein A1 is Asp or Asn, A2 is Met or Leu, A3 is Val or Leu, A4 is Asp or Glu and A5 is Asp or Glu.

2. The antibody of claim 1, wherein A1 is Asp, A2 is Met, A3 is Val, A4 is Asp and A5 is Asp.

3. The antibody of claim 1, wherein A1 is Asn, A2 is Leu, A3 is Leu, A4 is Glu and A5 is Glu.

4. The antibody of claim 1 or 2, wherein the antibody binds to tau comprising phospho-Ser422 and does not bind to tau comprising unphosphorylated Ser422.

5. The antibody according to any one of claims 1 to 4, wherein the antibody binds to MAP2 comprising phospho-Ser1808 and does not bind to MAP2 comprising unphosphorylated Ser1808.

6. The antibody of claim 2, wherein its Kd for the peptide consisting of Ser-Ile-Asp-Met-Val-Asp-Ser(PO₃H₂)-Pro-Gln-Leu-Ala-Thr-Leu-Ala-Asp is lower than 100nM.

7. The antibody according to any one of claims 1 to 6, wherein the antibody is monoclonal antibody.

8. The antibody according to any one of claims 1 to 7, wherein the antibody is produced by the hybridoma DSM ACC2762 or DSM ACC2763.

9. A method for diagnostic of neurological disorder, which method comprises
(a) contacting a sample with the antibody of claim 1 to 8; and
(b) detecting a complex formed between the antibody and tau or MAP2.

10. The method of claim 9, wherein neurological disorder is Alzheimer's disease.

11. A method for detecting tau comprising phospho-Ser422, which method comprises
(a) contacting a sample with the antibody of claim 1 to 8; and
(b) detecting a complex formed between the antibody and tau.

12. A method for detecting MAP2 comprising phospho-Ser1808, which method comprises
(a) contacting a sample with the antibody of claim 1 to 8; and
(b) detecting a complex formed between the antibody and MAP2.

13. The method of claim 11 or 12, wherein the complex is detected by western blotting, immunohistochemistry or ELISA.

14. Use of the antibody according to any one of claims 1 to 8 for diagnosis of neurological disorders.

15. Use of claim 15, wherein neurological disorder is Alzheimer's disease.

16. Use of the antibody according to any one of claim 1 to 8 for detecting tau comprising phospho-Ser422.

17. Use of the antibody according to any one of claims 1 to 8 for detecting MAP2 comprising phospho-Ser1808.

18. A kit for diagnosis of neurological disorders, which comprises the antibody according to any one of claims 1 to 8.

19. A kit of claim 18, wherein neurological disorder is Alzheimer's disease.

20. A kit for detecting tau comprising phosho-Ser422, which comprises the antibody according to any one of claims 1 to 8.

21. A kit for detecting MAP2 comprising phospho-Ser1808, which comprises the antibody according to any one of claim 1 to 8.

22. A cell which produces the antibody according to any one of claim 1 to 8.

23. The cell of claim 22, wherein the cell is hybridoma.

24. The cell of claim 23, wherein the hybridoma is DSM ACC2762 or DSM ACC2763.


SEQUENCE LISTING

<110>  F. Hoffmann-La Roche AG

<120>  An antibody which recognizes phosphorylated polypeptides

<130>  23732

<150>  EP06116550.2
<151>  2006-07-04

<160>  9

<170>  PatentIn version 3.3

<210>  1
<211>  441
<212>  PRT
<213>  Homo Sapiens

<400>  1

<210>  2
<211>  1827
<212>  PRT
<213>  Homo Sapiens

<400>  2
   Met Ala Asp Glu Arg Lys Asp Glu Gly Lys Ala Pro His Trp Thr Ser
   1               5                   10                  15
   Ala Pro Leu Thr Glu Ala Ser Ala His Ser His Pro Pro Glu Ile Lys
               20                  25                  30
   Asp Gln Gly Gly Ala Gly Glu Gly Leu Val Arg Ser Ala Asn Gly Phe
           35                  40                  45
   Pro Tyr Arg Glu Asp Glu Glu Gly Ala Phe Gly Glu His Gly Ser Gln
       50                  55                  60
   Gly Thr Tyr Ser Asn Thr Lys Glu Asn Gly Ile Asn Gly Glu Leu Thr
   65                  70                  75                  80
   Ser Ala Asp Arg Glu Thr Ala Glu Glu Val Ser Ala Arg Ile Val Gln
                   85                  90                  95
   Val Val Thr Ala Glu Ala Val Ala Val Leu Lys Gly Glu Gln Glu Lys
               100                 105                 110
   Glu Ala Gln His Lys Asp Gln Thr Ala Ala Leu Pro Leu Ala Ala Glu
           115                 120                 125
   Glu Thr Ala Asn Leu Pro Pro Ser Pro Pro Pro Ser Pro Ala Ser Glu
       130                 135                 140
   Gln Thr Val Thr Val Glu Glu Asp Leu Leu Thr Ala Ser Lys Met Glu
   145                 150                 155                 160
   Phe His Asp Gln Gln Glu Leu Thr Pro Ser Thr Ala Glu Pro Ser Asp
                   165                 170                 175
   Gln Lys Glu Lys Glu Ser Glu Lys Gln Ser Lys Pro Gly Glu Asp Leu
               180                 185                 190
   Lys His Ala Ala Leu Val Ser Gln Pro Glu Thr Thr Lys Thr Tyr Pro
           195                 200                 205
   Asp Lys Lys Asp Met Gln Gly Thr Glu Glu Glu Lys Ala Pro Leu Ala
       210                 215                 220
   Leu Phe Gly His Thr Leu Val Ala Ser Leu Glu Asp Met Lys Gln Lys
   225                 230                 235                 240
   Thr Glu Pro Ser Leu Val Val Pro Gly Ile Asp Leu Pro Lys Glu Pro
                   245                 250                 255
   Pro Thr Pro Lys Glu Gln Lys Asp Trp Phe Ile Glu Met Pro Thr Glu
               260                 265                 270
   Ala Lys Lys Asp Glu Trp Gly Leu Val Ala Pro Ile Ser Pro Gly Pro
   275                 280                 285
   Leu Thr Pro Met Arg Glu Lys Asp Val Phe Asp Asp Ile Pro Lys Trp
       290                 295                 300
   Glu Gly Lys Gln Phe Asp Ser Pro Met Pro Ser Pro Phe Gln Gly Gly
   305                 310                 315                 320
   Ser Phe Thr Leu Pro Leu Asp Val Met Lys Asn Glu Ile Val Thr Glu
                   325                 330                 335
   Thr Ser Pro Phe Ala Pro Ala Phe Leu Gln Pro Asp Asp Lys Lys Ser
               340                 345                 350
   Leu Gln Gln Thr Ser Gly Pro Ala Thr Ala Lys Asp Ser Phe Lys Ile
           355                 360                 365
   Glu Glu Pro His Glu Ala Lys Pro Asp Lys Met Ala Glu Ala Pro Pro
       370                 375                 380
   Ser Glu Ala Met Thr Leu Pro Lys Asp Ala His Ile Pro Val Val Glu
   385                 390                 395                 400
   Glu His Val Met Gly Lys Val Leu Glu Glu Glu Lys Glu Ala Ile Asn
                   405                 410                 415
   Gln Glu Thr Val Gln Gln Arg Asp Thr Phe Thr Pro Ser Gly Gln Glu
               420                 425                 430
   Pro Ile Leu Thr Glu Lys Glu Thr Glu Leu Lys Leu Glu Glu Lys Thr
           435                 440                 445
   Thr Ile Ser Asp Lys Glu Ala Val Pro Lys Glu Ser Lys Pro Pro Lys
       450                 455                 460
   Pro Ala Asp Glu Glu Ile Gly Ile Ile Gln Thr Ser Thr Glu His Thr
   465                 470                 475                 480
   Phe Ser Glu Gln Lys Asp Gln Glu Pro Thr Thr Asp Met Leu Lys Gln
                   485                 490                 495
   Asp Ser Phe Pro Val Ser Leu Glu Gln Ala Val Thr Asp Ser Ala Met
               500                 505                 510
   Thr Ser Lys Thr Leu Glu Lys Ala Met Thr Glu Pro Ser Ala Leu Ile
           515                 520                 525
   Glu Lys Ser Ser Ile Gln Glu Leu Phe Glu Met Arg Val Asp Asp Lys
       530                 535                 540
   Asp Lys Ile Glu Gly Val Gly Ala Ala Thr Ser Ala Glu Leu Asp Met
   545                 550                 555                 560
   Pro Phe Tyr Glu Asp Lys Ser Gly Met Ser Lys Tyr Phe Glu Thr Ser
   565 570 575
   Ala Leu Lys Glu Glu Ala Thr Lys Ser Ile Glu Pro Gly Ser Asp Tyr
   580 585 590
   Tyr Glu Leu Ser Asp Thr Arg Glu Ser Val His Glu Ser Ile Asp Thr
   595 600 605
   Met Ser Pro Met His Lys Asn Gly Asp Lys Glu Phe Gln Thr Gly Lys
   610 615 620
   Glu Ser Gln Pro Ser Pro Pro Ala Gln Glu Ala Gly Tyr Ser Thr Leu
   625 630 635 640
   Ala Gln Ser Tyr Pro Ser Asp Leu Pro Glu Glu Pro Ser Ser Pro Gln
   645 650 655
   Glu Arg Met Phe Thr Ile Asp Pro Lys Val Tyr Gly Glu Lys Arg Asp
   660 665 670
   Leu His Ser Lys Asn Lys Asp Asp Leu Thr Leu Ser Arg Ser Leu Gly
   675 680 685
   Leu Gly Gly Arg Ser Ala Ile Glu Gln Arg Ser Met Ser Ile Asn Leu
   690 695 700
   Pro Met Ser Cys Leu Asp Ser Ile Ala Leu Gly Phe Asn Phe Gly Arg
   705 710 715 720
   Gly His Asp Leu Ser Pro Leu Ala Ser Asp Ile Leu Thr Asn Thr Ser
   725 730 735
   Gly Ser Met Asp Glu Gly Asp Asp Tyr Leu Pro Ala Thr Thr Pro Ala
   740 745 750
   Leu Glu Lys Ala Pro Cys Phe Pro Val Glu Ser Lys Glu Glu Glu Gln
   755 760 765
   Ile Glu Lys Val Lys Ala Thr Gly Glu Glu Ser Thr Gln Ala Glu Ile
   770 775 780
   Ser Cys Glu Ser Pro Phe Leu Ala Lys Asp Phe Tyr Lys Asn Gly Thr
   785 790 795 800
   Val Met Ala Pro Asp Leu Pro Glu Met Leu Asp Leu Ala Gly Thr Arg
   805 810 815
   Ser Arg Leu Ala Ser Val Ser Ala Asp Ala Glu Val Ala Arg Arg Lys
   820 825 830
   Ser Val Pro Ser Glu Thr Val Val Glu Asp Ser Arg Thr Gly Leu Pro
   835                 840                 845
   Pro Val Thr Asp Glu Asn His Val Ile Val Lys Thr Asp Ser Gln Leu
       850                 855                 860
   Glu Asp Leu Gly Tyr Cys Val Phe Asn Lys Tyr Thr Val Pro Leu Pro
   865                 870                 875                 880
   Ser Pro Val Gln Asp Ser Glu Asn Leu Ser Gly Glu Ser Gly Thr Phe
                   885                 890                 895
   Tyr Glu Gly Thr Asp Asp Lys Val Arg Arg Asp Leu Ala Thr Asp Leu
               900                 905                 910
   Ser Leu Ile Glu Val Lys Leu Ala Ala Ala Gly Arg Val Lys Asp Glu
           915                 920                 925
   Phe Ser Val Asp Lys Glu Ala Ser Ala His Ile Ser Gly Asp Lys Ser
       930                 935                 940
   Gly Leu Ser Lys Glu Phe Asp Gln Glu Lys Lys Ala Asn Asp Arg Leu
   945                 950                 955                 960
   Asp Thr Val Leu Glu Lys Ser Glu Glu His Ala Asp Ser Lys Glu His
                   965                 970                 975
   Ala Lys Lys Thr Glu Glu Ala Gly Asp Glu Ile Glu Thr Phe Gly Leu
               980                 985                 990
   Gly Val Thr Tyr Glu Gln Ala Leu  Ala Lys Asp Leu Ser  Ile Pro Thr
           995                 1000                1005
   Asp Ala  Ser Ser Glu Lys Ala  Glu Lys Gly Leu Ser  Ser Val Pro
       1010                1015                1020
   Glu Ile  Ala Glu Val Glu Pro  Ser Lys Lys Val Glu  Gln Gly Leu
       1025                1030                1035
   Asp Phe  Ala Val Gln Gly Gln  Leu Asp Val Lys Ile  Ser Asp Phe
       1040                1045                1050
   Gly Gln  Met Ala Ser Gly Leu  Asn Ile Asp Asp Arg  Arg Ala Thr
       1055                1060                1065
   Glu Leu  Lys Leu Glu Ala Thr  Gln Asp Met Thr Pro  Ser Ser Lys
       1070                1075                1080
   Ala Pro  Gln Glu Ala Asp Ala  Phe Met Gly Val Glu  Ser Gly His
       1085                1090                1095
   Met Lys  Glu Gly Thr Lys Val  Ser Glu Thr Glu Val  Lys Gln Lys
       1100                1105                1110
   Val Ala Lys Pro Asp Leu Val His Gln Glu Ala Val Asp Lys Glu
       1115                1120                1125
   Glu Ser Tyr Glu Ser Ser Gly Glu His Glu Ser Leu Thr Met Glu
       1130                1135                1140
   Ser Leu Lys Ala Asp Glu Gly Lys Lys Glu Thr Ser Pro Glu Ser
       1145                1150                1155
   Ser Leu Ile Gln Asp Glu Ile Ala Val Lys Leu Ser Val Glu Ile
       1160                1165                1170
   Pro Cys Pro Pro Ala Val Ser Glu Ala Asp Leu Ala Thr Asp Glu
       1175                1180                1185
   Arg Ala Asp Val Gln Met Glu Phe Ile Gln Gly Pro Lys Glu Glu
       1190                1195                1200
   Ser Lys Glu Thr Pro Asp Ile Ser Ile Thr Pro Ser Asp Val Ala
       1205                1210                1215
   Glu Pro Leu His Glu Thr Ile Val Ser Glu Pro Ala Glu Ile Gln
       1220                1225                1230
   Ser Glu Glu Glu Glu Ile Glu Ala Gln Gly Glu Tyr Asp Lys Leu
       1235                1240                1245
   Leu Phe Arg Ser Asp Thr Leu Gln Ile Thr Asp Leu Gly Val Ser
       1250                1255                1260
   Gly Ala Arg Glu Glu Phe Val Glu Thr Cys Pro Ser Glu His Lys
       1265                1270                1275
   Gly Val Ile Glu Ser Val Val Thr Ile Glu Asp Asp Phe Ile Thr
       1280                1285                1290
   Val Val Gln Thr Thr Thr Asp Glu Gly Glu Ser Gly Ser His Ser
       1295                1300                1305
   Val Arg Phe Ala Ala Leu Glu Gln Pro Glu Val Glu Arg Arg Pro
       1310                1315                1320
   Ser Pro His Asp Glu Glu Glu Phe Glu Val Glu Glu Ala Ala Glu
       1325                1330                1335
   Ala Gln Ala Glu Pro Lys Asp Gly Ser Pro Glu Ala Pro Ala Ser
       1340                1345                1350
   Pro Glu Arg Glu Glu Val Ala Leu Ser Glu Tyr Lys Thr Glu Thr
       1355                1360                1365
   Tyr Asp Asp Tyr Lys Asp Glu Thr Thr Ile Asp Asp Ser Ile Met
   1370                    1375                    1380
   Asp Ala  Asp Ser Leu Trp Val  Asp Thr Gln Asp Asp  Asp Arg Ser
       1385                    1390                    1395
   Ile Met  Thr Glu Gln Leu Glu  Thr Ile Pro Lys Glu  Glu Lys Ala
       1400                    1405                    1410
   Glu Lys  Glu Ala Arg Arg Ser  Ser Leu Glu Lys His  Arg Lys Glu
       1415                    1420                    1425
   Lys Pro  Phe Lys Thr Gly Arg  Gly Arg Ile Ser Thr  Pro Glu Arg
       1430                    1435                    1440
   Lys Val  Ala Lys Lys Glu Pro  Ser Thr Val Ser Arg  Asp Glu Val
       1445                    1450                    1455
   Arg Arg  Lys Lys Ala Val Tyr  Lys Lys Ala Glu Leu  Ala Lys Lys
       1460                    1465                    1470
   Thr Glu  Val Gln Ala His Ser  Pro Ser Arg Lys Phe  Ile Leu Lys
       1475                    1480                    1485
   Pro Ala  Ile Lys Tyr Thr Arg  Pro Thr His Leu Ser  Cys Val Lys
       1490                    1495                    1500
   Arg Lys  Thr Thr Ala Ala Gly  Gly Glu Ser Ala Leu  Ala Pro Ser
       1505                    1510                    1515
   Val Phe  Lys Gln Ala Lys Asp  Lys Val Ser Asp Gly  Val Thr Lys
       1520                    1525                    1530
   Ser Pro  Glu Lys Arg Ser Ser  Leu Pro Arg Pro Ser  Ser Ile Leu
       1535                    1540                    1545
   Pro Pro  Arg Arg Gly Val Ser  Gly Asp Arg Asp Glu  Asn Ser Phe
       1550                    1555                    1560
   Ser Leu  Asn Ser Ser Ile Ser  Ser Ser Ala Arg Arg  Thr Thr Arg
       1565                    1570                    1575
   Ser Glu  Pro Ile Arg Arg Ala  Gly Lys Ser Gly Thr  Ser Thr Pro
       1580                    1585                    1590
   Thr Thr  Pro Gly Ser Thr Ala  Ile Thr Pro Gly Thr  Pro Pro Ser
       1595                    1600                    1605
   Tyr Ser  Ser Arg Thr Pro Gly  Thr Pro Gly Thr Pro  Ser Tyr Pro
       1610                    1615                    1620
   Arg Thr  Pro His Thr Pro Gly  Thr Pro Lys Ser Ala  Ile Leu Val
       1625                    1630                    1635
   Pro Ser Glu Lys Lys Val Ala Ile Ile Arg Thr Pro Pro Lys Ser
   1640 1645 1650
   Pro Ala Thr Pro Lys Gln Leu Arg Leu Ile Asn Gln Pro Leu Pro
   1655 1660 1665
   Asp Leu Lys Asn Val Lys Ser Lys Ile Gly Ser Thr Asp Asn Ile
   1670 1675 1680
   Lys Tyr Gln Pro Lys Gly Gly Gln Val Gln Ile Val Thr Lys Lys
   1685 1690 1695
   Ile Asp Leu Ser His Val Thr Ser Lys Cys Gly Ser Leu Lys Asn
   1700 1705 1710
   Ile Arg His Arg Pro Gly Gly Gly Arg Val Lys Ile Glu Ser Val
   1715 1720 1725
   Lys Leu Asp Phe Lys Glu Lys Val Gln Ala Lys Val Gly Ser Leu
   1730 1735 1740
   Asp Asn Ala His His Val Pro Gly Gly Gly Asn Val Lys Ile Asp
   1745 1750 1755
   Ser Gln Lys Leu Asn Phe Arg Glu His Ala Lys Ala Arg Val Asp
   1760 1765 1770
   His Gly Ala Glu Ile Ile Thr Gln Ser Pro Gly Arg Ser Ser Val
   1775 1780 1785
   Ala Ser Pro Arg Arg Leu Ser Asn Val Ser Ser Ser Gly Ser Ile
   1790 1795 1800
   Asn Leu Leu Glu Ser Pro Gln Leu Ala Thr Leu Ala Glu Asp Val
   1805 1810 1815
   Thr Ala Ala Leu Ala Lys Gln Gly Leu
   1820 1825

<210>  3
<211>  16
<212>  PRT
<213>  Artificial Sequence

<220>
<223>  Sensitized antigen peptide

<220>
<221>  MOD_RES
<222>  (8)..(8)
<223>  PHOSPHORYLATION

<400>  3

Cys Ser Ile Asp Met Val Asp Ser Pro Gln Leu Ala Thr Leu Ala Asp

1               5                   10                  15


<210>  4
<211>  15
<212>  PRT
<213>  Homo sapiens

<400>  4

Ser Ile Asp Met Val Asp Ser Pro Gln Leu Ala Thr Leu Ala Asp
1               5                   10                  15


<210>  5
<211>  15
<212>  PRT
<213>  Homo sapiens

<400>  5

Ser Ile Asn Leu Leu Glu Ser Pro Gln Leu Ala Thr Leu Ala Glu
1               5                   10                  15


<210>  6
<211>  15
<212>  PRT
<213>  Artificial Sequence

<220>
<223>  phosphorylated peptide


<220>
<221>  MOD_RES
<222>  (7)..(7)
<223>  PHOSPHORYLATION

<400>  6

Ser Ile Asp Met Val Asp Ser Pro Gln Leu Ala Thr Leu Ala Asp
1               5                   10                  15


<210>  7
<211>  15
<212>  PRT
<213>  Artificial Sequence

<220>
<223>  phosphorylated peptide


<220>
<221>  MOD_RES
<222>  (7)..(7)
<223>  PHOSPHORYLATION

<400>  7

Ser Ile Asn Leu Leu Glu Ser Pro Gln Leu Ala Thr Leu Ala Glu
1               5                   10                  15


<210>  8
<211>  15
<212>  PRT
<213>  Artificial Sequence

<220>
<223>  consensus sequence


<220>
<221>  VARIANT
<222>  (3)..(3)
<223>  Xaa =Asp or Asn

<220>
<221>  VARIANT
<222>  (4)..(4)
<223>  Xaa =Met or Leu

<220>
<221>  VARIANT
<222>  (5)..(5)
<223>  Xaa =Val or Leu

<220>
<221>  VARIANT
<222>  (6)..(6)
<223>  Xaa =Asp or Glu

<220>
<221>  VARIANT
<222>  (15)..(15)
<223>  Xaa =Asp or Glu

<400>  8

Ser Ile Xaa Xaa Xaa Xaa Ser Pro Gln Leu Ala Thr Leu Ala Xaa
1               5                   10                  15


<210>  9
<211>  15
<212>  PRT
<213>  Artificial Sequence

<220>
<223>  consensus sequence


<220>
<221>  VARIANT
<222>  (3)..(3)
<223>  Xaa =Asp or Asn

<220>
<221>  VARIANT
<222>  (4)..(4)
<223>  Xaa =Met or Leu

<220>
<221>  VARIANT
<222>  (5)..(5)
<223>  Xaa =Val or Leu

<220>
<221>  VARIANT
<222>  (6)..(6)
<223>  Xaa =Asp or Glu

<220>
<221>  MOD_RES
<222>  (7)..(7)
<223>  PHOSPHORYLATION

<220>

<221>  VARIANT
<222>  (15)..(15)
<223>  Xaa =Asp or Glu

<400>  9

Ser Ile Xaa Xaa Xaa Xaa Ser Pro Gln Leu Ala Thr Leu Ala Xaa
1               5                   10                  15